# EUROPEAN PATENT APPLICATION

(11) **EP 2 682 392 A2**
(43) Date of publication of application: **08.01.2014**
(21) Application number: 12752567.3
(22) Date of filing: 27.02.2012
(51) Int. Cl.: C07D 401/04, A61K 31/454, A61P 19/10

(54) **PYRAZOLE DERIVATIVES, PREPARATION METHOD THEREOF, AND COMPOSITION FOR PREVENTING AND TREATING OSTEOPOROSIS CONTAINING SAME**

(30) Priority: 28.02.2011 KR 20110018037
(71) Applicant: EWHA University-Industry Collaboration Foundation, Seodaemun-gu Seoul 120-750 (KR)
(72) Inventor: BAE, Yun Soo, Goyang-si Gyeonggi-do 410-904 (KR); LEE, Kee In, Daejeon 305-755 (KR); LEE, Jee Hyun, Seoul 138-240 (KR); LEE, Soo Young, Seoul 135-778 (KR); CHOI, Sun, Seoul 140-120 (KR)
(74) Representative: McConchie, Connor Fraser
(86) International application number: PCT/KR2012/001457
(87) International publication number: WO 2012/118309

(57) **Abstract**

The present invention provides a pyrazole derivative compound and a pharmaceutically acceptable salt thereof. The compound of the invention is remarkably effective for preventing and treating osteoporosis.

## Description

### [Technical Field]

The present invention relates to a novel pyrazole derivative having excellent inhibitory activity against reactive oxygen species, a preparation method thereof, and a composition comprising the same for preventing and treating osteoporosis.

### [Background Art]

The process of bone modeling and remodeling plays an important role in development, growth and metabolism of bone. Bone modeling initiates from birth and then continues until adolescence/manhood at which time the skeleton matures to an end of growth of an individual, thus achieving the peak bone mass from between his/her twenties to early-thirties. Since then, a bone remodeling process involving removal and replacement of bone is repeated for about 3 years, during which bone formation and bone resorption are coupled to maintain the balance therebetween. After this period of time, bone formation cannot sufficiently keep up with bone loss occurring due to bone resorption, which eventually results in an about 0.3 to 0.5% annual decrease in bone mass. In particular, women will undergo a significant bone loss of 2 to 3% yearly at the early stage of menopause.

Bone contains four types of cells such as osteoblasts osteoclasts, lining cells, and osteocytes. Here, osteoblasts, which are derived from bone marrow stromal cells, are differentiated cells of synthesizing a bone matrix and play a leading part in bone formation, whereas osteoclasts, which are derived from hematopoietic stem cells, play a leading part in bone resorption.

Osteoporosis is a condition in which the compact bone becomes thinner as the calcified bone tissue density decreases and thus the bone-marrow cavity becomes larger. Accordingly as the condition develops, the bones become fragile and are thus prone to fracture even with a small impact. Bone mass is affected by various factors such as genetics, nutrition, changes in hormone levels, differences in exercise and lifestyle, etc., and it is known that the main causes of osteoporosis are aging, insufficient exercise, being underweight, smoking, low-calcium dietary intake, menopause, ovariectomy, etc. Meanwhile, although there are individual differences, the bone resorption level of black people is lower than that of white people, and thus their bone mass is higher than that of white people. For most people, bone mass peaks at ages of 14 to 18 and decreases about 1% per year at old ages. Particularly, in the case of women, bone loss occurs continuously after the age of 30 and develops rapidly due to a change in hormone balance during menopause. That is, when a woman reaches menopause, the estrogen level decreases rapidly. At this time, large numbers of B-lymphocytes are formed as if it happened by interleukin=7 (IL-7), and pre-B cells are accumulated in bone marrow, which consequently leads to an increase in the level of IL-6, thus resulting in an increased activity of osteoclasts and finally a decreased level of bone mass.

As such, although there are differences in the degree of osteoporosis, it is an inevitable condition for old people, particularly for women after menopause. Thus, in the developed countries, the interest in osteoporosis and its therapeutic agents is increasing as the population is aging. Moreover, it is known that a market of about 130 billion dollars is formed worldwide in the treatment of bone diseases and is expected to further increase. Thus, global research institutes and pharmaceutical companies have invested heavily in the development of therapeutic agents for bone diseases. Also, the prevalence rate of osteoporosis in recently Korea increases rapidly as the average life expectancy approaches 80 years. According to research recently conducted for local residents, when the research results are normalized in terms of total national population, it has been reported that 4.5% of males have osteoporosis and 19.8% of females suffer from the same disease. This suggests that osteoporosis is a more common disease than diabetes or cardiovascular diseases and is a very important public health issue in view of the pain of patients due to fracture or the cost incurred for the treatment.

Until now, various substances have been developed as therapeutic agents for osteoporosis. Among those therapeutic substances, estrogen, which is most commonly used as an therapeutic agents for osteoporosis but whose practical efficacy has not yet been demonstrated, disadvantageously requires life-time administration, and long-term administration thereof may result in adverse side effects such as increased risk of breast cancer or uterine cancer. Alendronate also has problems associated with indefinite understanding of medicinal efficacy, sluggish gastrointestinal absorption, and pathogenesis of inflammation on gastrointestinal and esophageal mucosa. The calcium preparation is known to have fewer side effects and excellent efficacy, but it is a preventive agent rather than a therapeutic agent. Moreover, vitamin D preparations such as calcitonin are known, but their efficacy and side effects have not yet been sufficiently studied. Thus, there is a need for the development of a novel therapeutic agent for metabolic bone diseases, which has fewer side effects and excellent efficacy.

Meanwhile, there by recently been studies reporting that reactive oxygen species (ROS) caused by oxidative stress are involved in bone metabolism (Darden, A. G., et al., J. Bone Miner, Res., 11:671-675, 1996; Yang, S., et al., J. Biol. Chem., 276:5452-5458, 2001; Fraser, J. H., et al., Bone 19:223-226, 1996; and Yang, S., et al., Calcif. Tissue Int., 63:346-350, 1998). Moreover, it is known that bone remodeling is carried out through the relative action between bone-forming osteoblasts and bone-resorbing osteoclasts (OC). Multinuclear osteoclasts differentiate from the monocyte/macrophage lineage of hematopoietic progenitor cells through a multi-stage process of cell adhesion, proliferation, motility, cell-cell contact, and terminal fusion for the formation of multinucleated giant cells. This process is initiated by binding of a receptor activator of nuclear factor-kB (hereinafter referred to as "RANK") to a receptor activator of nuclear factor-kB ligand (hereinafter referred to as "RANKL") and is then transmitted through the activation of several signaling cascades. The activated signaling pathway includes NF-KB, extracellular signal-regulated kinase (hereinafter referred to as "ERK"), c-Jun N-terminal kinase (hereinafter referred to as "JNK"), and p38 mitogen-activated protein (MAP) kinase through tumor necrosis factor (TNF) receptor-associated factor 6 (hereinafter referred to as "TRAF6"). These signaling events have a direct effect on the modulation of differentiation and action of osteoclasts (Boyle, N. J., et al., Nature, 423:337-342, 2003). Once osteoclasts are differentiated, the resorption of bone is accelerated by ROS generated due to nicotinamide adenine dinucleotide phosphate (NADPH) oxidase. An NADPH oxidase inhibitor leads to a reduction of ROS and bone resorption (Yang, S., et al., Calcif. Tissue Int., 63:346-350, 1998). These results are consistent with the theory suggesting that the generation of ROS in osteoclasts is dependent on the activity of NADPH oxidase and is directly associated with the function of osteoclasts.

Therefore, the inventors of the present invention have conducted extensive studies based on the fact that a therapeutic agent for osteoporosis can be developed using a molecular mechanism which inhibits the generation of reactive oxygen species (ROS) and found that pyrazole derivatives of the present invention exhibit excellent inhibitory activity on the generation of ROS and these compounds can be used for the prevention or treatment of osteoporosis, thereby completing the present invention.

### [Disclosure]

### [Technical Problem]

An object of the present invention is to provide a novel pyrazole derivative having excellent inhibitory activity on the generation of reactive oxygen species, a preparation method thereof, and a composition comprising the same for the treatment of osteoporosis.

Another object of the present invention is to provide a method for preventing or treating osteoporosis, the method comprising administering a novel pyrazole derivative of the present invention to a subject in need thereof, and a use of the novel pyrazole derivative of the present invention for preparing a pharmaceutical preparation for the prevention or treatment of osteoporosis.

### [Technical Solution]

The present invention provides a compound represented by the following formula 1 or a pharmaceutically acceptable salt thereof: 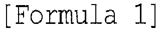 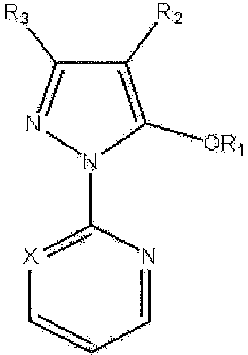 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₁ and R₂ each represents a hydrogen atom, R₃ represents a phenyl, a nitrophenyl, or a substituted or unsubstituted diphenylethenyl; and
wherein the substituent is a nitro, a hydroxyl, or a methoxyl.

In the compound represented by formula 1 or a pharmaceutically acceptable salt thereof of the present invention, R₂ may preferably represent a hydrogen atom, a propyl, or a benzyl, and R₃ may preferably represent a phenyl, a nitrophenyl, or a 1,2-diphenylethenyl.

In the compound represented by formula 1, when X represents -CH- and R₁ and R₂ each represents a hydrogen atom, R₃ may preferably represent a substituted or unsubstituted diphenylethenyl, or when X represents a nitrogen atom and R₁ and R₂ each represents a hydrogen atom, R₃ may preferably represent a nitrophenyl or a substituted or unsubstituted diphenylethenyl. In the salt of the compound represent by formula 1, when R₁ and R₂ each represents a hydrogen atom, R₃ may preferably represent a phenyl, a nitrophenyl, or a substituted or unsubstituted diphenylethenyl.

The compound of the present invention may preferably be a compound selected from:
1-(pyridin-2-yl)-3-phenyl-4-propyl-1*H*-pyrazol-5-ol,
1-(pyridin-2-yl-3-phenyl-4-benzyl-1*H*-pyrazol-5-ol,
1-(pyridin-2-yl)-3-phenyl-4-(4-nitrobenzyl)-1*H*-pyrazol-5-ol,
1-(pyridin-2-yl)-3-phenyl-4-propyl-1*H*-pyrazol-5-ol hydrochloride,
1-(pyridin-2-yl)-3-phenyl-4-propyl-1*H*-5-(isopropyloxycarbonyloxymethyloxy)pyrazole,
1-(pyridin-2-yl)-3-[(3-methoxy-4-hydroxyphenyl)-E-ethenyl]-4-benzyl-1*H*-pyrazol-5-ol hydrochloride,
1-(pyridin-2-yl)-3-[(3-methoxy-4-hydroxyphenyl)-E-ethenyl]-4-benzyl-1*H*-pyrazol-5-ol,
3-(4-nitrophenyl)-3-[(pyrimidin-2-yl)hydrazono]-propionic acid ethyl ester,
1-(pyrimidin-2-yl)-3-(4-nitrophenyl)-1H-pyrazol-5-ol,
1-(pyridin-2-yl)-3-(1,2-diphenyl-E-ethenyl)-1H-pyrazol-5-ol,
1-(pyridin-2-yl)-3-phenyl-1H-pyrazol-5-ol hydrochloride;
3-(4-methoxyphenylamino)-1H-pyrazol-5-ol, or
4-hexylidene-3-phenyl-1-(pyridin-2-yl)-1H-pyrazol-5-ol;
and a pharmaceutically acceptable salt thereof.

The compound of the present invention may preferably be a compound selected from:
1-(pyridin-2-yl)-3-phenyl-4-propyl-1*H*-pyrazol-5-ol hydrochloride,
1-(pyridin-2-yl)-3-phenyl-4-propyl-1*H*-5-(isopropyloxycarbonyloxymethyloxy)pyrazole,
1-(pyridin-2-yl)-3-[(3-methoxy-4-hydroxyphenyl)-E-ethenyl]-4-benzyl-1*H*-pyrazol-5-ol,
1-(pyridin-2-yl)-3-[(3-methoxy-4-hydroxyphenyl)-E-ethenyl]-4-benzyl-1*H*-pyrazol-5-ol hydrochloride,
1-(pyridin-2-yl)-3-phenyl-1H-pyrazol-5-ol hydrochloride;
   and a pharmaceutically acceptable salt thereof.

As used herein, the term "pharmaceutically acceptable salt" refers to a salt commonly used in the pharmaceutical industry, and examples thereof may be a salt of inorganic acid prepared using hydrochloric acid, nitric acid, phosphoric acid, bromic acid, iodic acid, perchloric acid, tartaric acid, sulfuric acid, etc.; a salt of organic acid prepared using acetic acid, trifluoroacetic acid, citric acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, etc.; and a salt of sulfonic acid prepared using methanesulfonic acid, ethanesulfonic acid, benzenesulfonic acid, p-toluenesulfonic acid, naphthalene sulfonic acid, etc.. However, the pharmaceutically acceptable salt of the present invention is not limited thereto. The pharmaceutically acceptable sale may preferably be a hydrochloric acid salt.

The compound represented by formula 1, the above-mentioned compound, or a pharmaceutically acceptable salt thereof of the present invention can prevent or treat osteoporosis by inhibiting the generation of reactive oxygen species. For example, the compound represented by formula 1, the above-mentioned compound, or a pharmaceutically acceptable salt thereof of the present invention can inhibit the generation of reactive oxygen species by inhibiting NADPH oxidase. The production of osteoclasts can be inhibited by the inhibition of NADPH oxidase which is an important substance for differentiation of macrophages into osteoclasts.

The compound represented by formula 1, the above-mentioned compound, or a pharmaceutically acceptable salt thereof of the present invention can prevent or treat osteoporosis by inhibiting the generation of reactive oxygen species. For example, the compound represented by formula 1, the above-mentioned compound, or a pharmaceutically acceptable salt thereof of the present invention can inhibit the generation of reactive oxygen species by suppressing NADPH oxidase.

The compound represented by formula 1, the above-mentioned compound, or a pharmaceutically acceptable salt thereof of the present invention can inhibit the production of osteoclasts by inhibiting NADPH oxidase which is an important substance for differentiation of macrophages into osteoclasts.

The compound represented by formula 1, the above-mentioned compound, or a pharmaceutically acceptable salt thereof of the present invention can treat or prevent osteoporosis by inhibiting bone destruction.

The compound represented by formula 1, the above-mentioned compound, or a pharmaceutically acceptable salt thereof of the present invention have low toxicity and are not decomposed in the blood for a long time due to excellent stability, thereby maintaining high levels in the blood for a long time. That is, the compound represented by formula 1, the above-mentioned compound, or a pharmaceutically acceptable salt thereof of the present invention exhibit significantly low toxicity and significantly high stability compared to those of the compound disclosed in Korean Patent No. KR 0942382, thereby being capable of exhibiting excellent effects in the treatment or prevention of osteoporosis.

The present invention provides a method for preparing a compound represented by the following formula 1 or a pharmaceutically acceptable salt thereof, the method comprising:
adding dropwise a compound represented by the following formula 2 and a compound represented by the following formula 3 to a polar organic solvent; and
heating the polar organic solvent comprising the compound represented by formula 2 and the compound represented by formula 3: 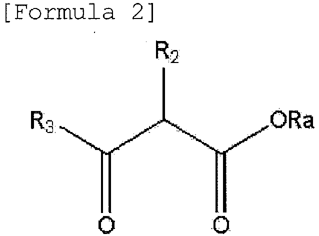 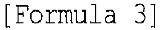 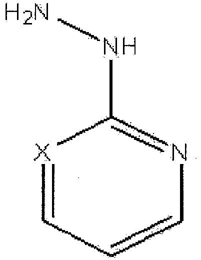 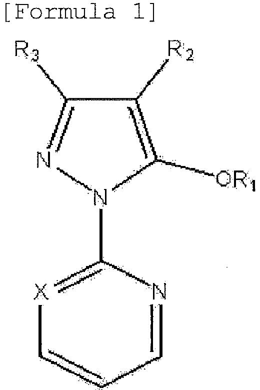
wherein X represents -CH- or nitrogen;
Ra represents a C1-C4 linear or branched alkyl;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₁ and R₂ each represents a hydrogen atom, R₃ represents a phenyl, a nitrophenyl, or a substituted or unsubstituted diphenylethenyl; and
wherein the substituent is a nitro, a hydroxyl, or a methoxyl.

According to the preparation method of the present invention, the following compound:
1-(pyridin-2-yl)-3-phenyl-4-propyl-1*H*-pyrazol-5-ol,
1-(pyridin-2-yl)-3-phenyl-4-benzyl-1*H*-pyrazol-5-ol,
1-(pyridin-2-yl)-3-phenyl-4-(4-nitrobenzyl)-1*H*-pyrazol-5-ol,
1-(pyridin-2-yl)-3-phenyl-4-propyl-1*H*-pyrazol-5-ol hydrochloride,
1-(pyridin-2-yl)-3-phenyl-4-propyl-1*H*-5-(isopropyloxycarbonyloxymethyloxy)pyrazole,
1-(pyridin-2-yl)-3-[(3-methoxy-4-hydroxyphenyl)-E-ethenyl]-4-benzyl-1*H*-pyrazol-5-ol hydrochloride,
1-(pyridin-2-yl)-3-[(3-methoxy-4-hydroxyphenyl)-E-ethenyl]-4-benzyl-1*H*-pyrazol-5-ol,
3-(4-nitrophenyl)-3-[(pyrimidin-2-yl)hydrazono]-propionic acid ethyl ester,
1-(pyrimidin-2-yl)-3-(4-nitrophenyl)-1*H*-pyrazol-5-ol,
1-(pyridin-2-yl)-3-(1,2-diphenyl-E-ethenyl)-1*H*-pyrazol-5-ol,
1-(pyridin-2-yl)-3-phenyl-1*H*-pyrazol-5-ol hydrochloride;
3-(4-methoxyphenylamino)-1*H*-pyrazol-5-ol, or
4-hexylidene-3-phenyl-1-(pyridin-2-yl)-1*H*-pyrazol-5-ol;
or a pharmaceutically acceptable salt thereof can be prepared.

In the preparation method of the present invention, the following compound:
1-(pyridin-2-yl)-3-phenyl-4-propyl-1*H*-pyrazol-5-ol hydrochloride;
1-(pyridin-2-yl)-3-phenyl-4-propyl-1*H*-5-(isopropyloxycarbonyloxymethyloxy)pyrazole;
1-(pyridin-2-yl)-3-[(3-methoxy-4-hydroxyphenyl)-E-ethenyl]-4-benzyl-1*H*-pyrazol-5-ol hydrochloride;
1-(pyridin-2-yl)-3-[(3-methoxy-4-hydroxyphenyl)-E-ethenyl]-4-benzyl-1H-pyrazol-5-ol; or
1-(pyridin-2-yl)-3-phenyl-*1H*-pyrazol-5-ol hydrochloride;
or a pharmaceutically acceptable salt thereof can preferably be prepared.

In the compound represented by formula 1, the above-mentioned compound, or a pharmaceutically acceptable salt thereof of the present invention, α-substituted β-keto ester, which is the compound represented by formula 2 used as a starting material, is commercially available or can be prepared according to the method described in J. Org. Chem., Vol. 43, No. 10, 1978, 2087-2088, specifically by reacting a commercially available acyl chloride derivative with Meldrum's acid and heating the resulting product under reflux in the presence of an organic solvent such as methanol or ethanol to form β-keto ester. The α-substituted β-keto ester can be prepared according to the method described in J. Chem. Soc., Perkin Trans. 1, 1986, 1139-1143. More specifically, it can be easily prepared by reaction of β-keto ester with alkyl halide in the presence of potassium carbonate or cesium carbonate.

In the preparation method of the present invention, the compound represented by formula 3 as a reactant may be commercially available and may be used in an amount of about 1 to 3 molar equivalents, preferably about 1 to 1.3 molar equivalents, based on 1 molar equivalent of the compound represented by formula 2 as a starting material.

In the preparation method of the present invention, the polar organic solvent may be selected from C1-C4 alcohol such as methanol, ethanol, n-propanol, i-isopropanol, n-butanol or t-butanol, acetic acid, or a mixture thereof and may preferably be ethanol or acetic acid.

In the preparation method of the present invention, the salt of the compound represented by formula 1 may be prepared by reacting the compound represented by formula 1 with an acid material. The acid material is not particularly limited as long as it can form a salt by reaction with the compound represented by formula 1. For example, the acid material may be inorganic acid such as hydrochloric acid, nitric acid, phosphoric acid, bromic acid, iodic acid, perchloric acid, tartaric acid, and sulfuric acid; organic acids such as acetic acid, trifluoroacetic acid, citric acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, and hydroiodic acid; and sulfonic acids such as methanesulfonic acid, ethanesulfonic acid, benzenesulfonic acid, p-toluenesulfonic acid, and naphthalene sulfonic acid. and may preferably be hydrochloric acid.

In the preparation method of the present invention, the compound represented by formula 2 and the compound represented by formula 3 may be added dropwise to a polar organic solvent at -4 °C to 10 °C.

In the preparation method of the present invention, the organic solvent comprising the compound represented by formula 2 and the compound represented by formula 3 may preferably be heated at a reflux temperature of the solvent, preferably at a temperature of about 100 to about 130 °C.

In the preparation method of the present invention, the organic solvent comprising the compound represented by formula 2 and the compound represented by formula 3 may preferably be heated for about 10 minutes to 72 hours.

In the preparation method of the present invention, a compound represented by formula 4 may be prepared by reaction of the compound represented by formula 2 and the compound represented by formula 3 in the presence of an organic solvent. 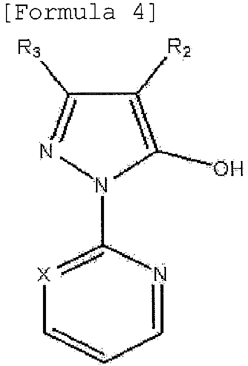 wherein X represents -CH- or nitrogen;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₂ represents a hydrogen atom, R₃ represents a phenyl, a nitrophenyl, or a substituted or unsubstituted diphenylethenyl; and
wherein the substituent is a nitro, a hydroxyl, or a methoxyl.

In the method for preparing the compound represented by the following formula 1, the compound represented by formula 4 may react with a halide compound such as isopropyloxycarbonyloxymethyliodide, isopropyloxycarbonyloxymethylchloride, or isopropyloxycarbonyloxymethylbromide in the presence of a base to prepare the compound represented by the following formula 1 comprising an isopropyloxycarbonyloxymethyloxy group bonded to the carbon at the 5 position of pyrazole.

In the reaction of the compound represented by formula 4 with the halide compound, the base may preferably be 4-dimethylaminopyridine (DMAP), pyridine, triethylamine, imidazole, a metal salt of carbonate such as carbonate potassium, carbonate sodium, or carbonate calcium, or a mixture thereof. The base may preferably be used in an amount of about 0.05 to 3 molar equivalents, preferably about 2 to 3 molar equivalents, based on 1 molar equivalent of the compound represented by formula 2.

In the reaction of the compound represented by formula 4 with the halide compound, the reaction solvent may preferably be a mixture of water and an organic solvent, preferably a mixture of water and at least one organic solvent selected from methylene chloride, ethyl ether, ethyl acetate, tetrahydrofuran (THF), and N,N'-dimethylformamide (DMF), more preferably, a mixture of water and methylene chloride.

The reaction of the compound represented by formula 4 with the halide compound may be carried out in the presence of a phase transfer catalyst. In this case, the compound represented by formula 1 can be obtained in higher purity by preventing the generation of impurities comprising an alkylated amine group at the 2 position of the pyrazole group. The phase transfer catalyst is not particularly limited, but it may preferably be Bu₄NHSO₄.

In the reaction of the compound represented by formula 4 with the halide compound, the reaction temperature may preferably be about 0 to 40 °C, more preferably 15 to 30 °C, and the reaction time may preferably be 10 to 12 hours. However, depending on the reaction rate, the reaction temperature may be further increased, and the reaction time may be further increased.

The present invention provides a pharmaceutical composition for preventing or treating osteoporosis, comprising a compound represented by the following formula 1 or a pharmaceutically acceptable salt thereof: 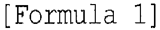 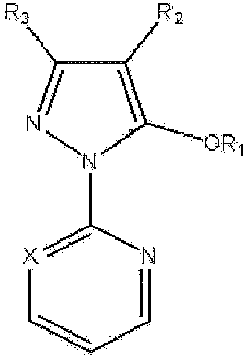 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₁ and R₂ each represents a hydrogen atom, R₃ represents a phenyl, a nitrophenyl, or a substituted or unsubstituted diphenylethenyl; and
wherein the substituent is a nitro, a hydroxyl, or a methoxyl.

In the compound represented by formula 1, when X represents -CH- and R₁ and R₂ each represents a hydrogen atom, R₃ may preferably represent a substituted or unsubstituted diphenylethenyl, or when X represents a nitrogen atom and R₁ and R₂ each represents a hydrogen atom, R₃ may preferably represent a nitrophenyl or a substituted or unsubstituted diphenylethenyl. In the salt of the compound represent by formula 1, when R₁ and R₂ each represents a hydrogen atom, R₃ may preferably represent a phenyl, a nitrophenyl, or a substituted or unsubstituted diphenylethenyl.

The present invention provides a composition for preventing or treating osteoporosis, comprising a compound, selected from:
1-(pyridin-2-yl)-3-phenyl-4-propyl-*1H-*pyrazol-5-ol,
1-(pyridin-2-yl)-3-phenyl-4-benzyl-1*H*-pyrazol-5-ol,
1-(pyridin-2-yl)-3-phenyl-4-(4-nitrobenzyl)-*1H*-pyrazol-5-ol,
1-(pyridin-2-yl)-3-phenyl-4-propyl-1*H*-pyrazol-5-ol hydrochloride,
1-(pyridin-2-yl)-3-phenyl-4-propyl-*1H-*5-(isopropyloxycarbonyloxymethyloxy)pyrazole,
1-(pyridin-2-yl)-3-[(3-methoxy-4-hydroxyphenyl)-E-ethenyl]-4-benzyl-*1H*-pyrazol-5-ol hydrochloride,
1-(pyridin-2-yl)-3-[(3-methoxy-4-hydroxyphenyl)-E-ethenyl]-4-benzyl-1*H*-pyrazol-5-ol,
3-(4-nitrophenyl)-3-[(pyrimidin-2-yl)hydrazono]-propionic acid ethyl ester,
1-(pyrimidin-2-yl)-3-(4-nitrophenyl)-*1H*-pyrazol-5-ol,
1-(pyridin-2-yl)-3-(1,2-diphenyl-*E*-ethenyl)-*1H*-pyrazol-5-ol,
1-(pyridin-2-yl)-3-phenyl-*1H*-pyrazol-5-ol hydrochloride;
3-(4-methoxyphenylamino)-1*H*-pyrazol-5-ol, or
4-hexylidene-3-phenyl-1-(pyridin-2-yl)-*1H*-pyrazol-5-ol; and a pharmaceutically acceptable salt thereof.

Preferably, the present invention provides a composition for preventing or treating osteoporosis, comprising at least one compound selected from:
1-(pyridin-2-yl)-3-phenyl-4-propyl-*1H*-pyrazol-5-ol hydrochloride,
1-(pyridin-2-yl)-3-phenyl-4-propyl-*1H*-5-(isopropyloxycarbonyloxymethyloxy)pyrazole,
1-(pyridin-2-yl)-3-[(3-methoxy-4-hydroxyphenyl)-E-ethenyl]-4-benzyl-1*H*-pyrazol-5-ol,
1-(pyridin-2-yl)-3-[(3-methoxy-4-hydroxyphenyl)-E-ethenyl]-4-benzyl-1*H*-pyrazol-5-ol hydrochloride, or
1-(pyridin-2-yl)-3-phenyl-*1H*-pyrazol-5-ol hydrochloride; or a pharmaceutically acceptable salt thereof.

As used herein, the term "osteoporosis" refers to a condition in which an absolute quantity of bone with exclusion of a vacant portion (such as marrow cavity) from the entire bone has been decreased, and includes senile osteoporosis, post-menopausal osteoporosis, endocrine osteoporosis, congenital osteoporosis, immobilized osteoporosis and post-traumatic osteoporosis.

The composition comprising the compound represented by formula 1, the above-mentioned compound, or a pharmaceutically acceptable salt thereof of the present invention can prevent or treat osteoporosis by inhibiting the generation of reactive oxygen species. For example, the composition comprising the compound represented by formula 1, the above-mentioned compound, or a pharmaceutically acceptable salt thereof of the present invention can inhibit the generation of reactive oxygen species by inhibiting NADPH oxidase. The composition of the present invention can inhibit the production of osteoclasts by the inhibition of NADPH oxidase which is an important substance for differentiation of macrophages into osteoclasts.

The composition comprising the compound represented by formula 1, the above-mentioned compound, or a pharmaceutically acceptable salt thereof of the present invention can treat or prevent osteoporosis by inhibiting bone destruction.

The composition comprising the compound represented by formula 1, the above-mentioned compound, or a pharmaceutically acceptable salt thereof of the present invention have low toxicity and are not decomposed in the blood for a long time due to excellent stability, thereby maintaining high levels in the blood for a long time. That is, the composition comprising the compound represented by formula 1, the above-mentioned compound, or a pharmaceutically acceptable salt thereof of the present invention exhibit significantly low toxicity and significantly high stability compared to those of the compound disclosed in Korean Patent No. KR 0942382, thereby being capable of exhibiting excellent effects in the treatment or prevention of osteoporosis.

The composition of the present invention may include pharmaceutically acceptable additives, such as a diluent, a binder, a disintegrant, a lubricant, a pH-adjusting agent, an antioxidant and a solubilizer, within the range where effects of the present invention are not impaired.

Examples of the diluent include sugar, starch, microcrystalline cellulose, lactose (lactose hydrate), glucose, D-mannitol, alginate, an alkaline earth metal salt, clay, polyethylene glycol, anhydrous dibasic calcium phosphate, and a mixture thereof; Examples of the binder include starch, microcrystalline cellulose, highly dispersive silica, mannitol, D-mannitol, sucrose, lactose hydrate, polyethylene glycol, polyvinylpyrrolidone (povidone), a polyvinylpyrrolidone copolymer (copovidone), hypromellose, hydroxypropylcellulose, natural gum, synthetic gum, copovidone, gelatin, and a mixture thereof.

Examples of the disintegrant include starches or modified starches such as sodium starch glycolate, corn starch, potato starch, and pregelatinized starch; clays such as bentonite, montmorillonite, and veegum; celluloses such as microcrystalline cellulose, hydroxypropylcellulose, and carboxymethylcellulose; algins such as sodium alginate, and alginic acid; crosslinked celluloses such as croscarmellose sodium; gums such as guar gum, and xanthan gum; crosslinked polymers such as crosslinked polyvinylpyrrolidone (crospovidone); effervescent agents such as sodium bicarbonate and citric acid, and mixtures thereof.

Examples of the lubricant include talc, stearic acid, magnesium stearate, calcium stearate, sodium lauryl sulfate, hydrogenated vegetable oil, sodium benzoate, sodium stearyl fumarate, glyceryl behenate, glyceryl monolaurate, glyceryl monostearate, glyceryl palmitostearate, colloidal silicon dioxide, and mixtures thereof.

Examples of the pH-adjusting agent include acidifying agents such as acetic acid, adipic acid, ascorbic acid, sodium ascorbate, sodium etherate, malic acid, succinic acid, tartaric acid, fumaric acid, and citric acid, and basifying agents such as precipitated calcium carbonate, aqueous ammonia, meglumine, sodium carbonate, magnesium oxide, magnesium carbonate, sodium citrate, and tribasic calcium phosphate.

Examples of the antioxidant include dibutyl hydroxy toluene, butylated hydroxyanisole, tocopherol acetate, tocopherol, propyl gallate, sodium hydrogen sulfite, and sodium pyrosulfite. Examples of the solubilizer that can be used in the immediate-release compartment of the present invention include sodium lauryl sulfate, polyoxyethylene sorbitan fatty acid ester (such as polysorbate), docusate sodium and poloxamer.

Moreover, In order to prepare a delayed-release formulation, the composition of the present invention may include an enteric polymer, a water-insoluble polymer, a hydrophobic compound, and a hydrophilic polymer.

The enteric polymer refers to a polymer which is insoluble or stable under acidic conditions of less than pH 5 and is dissolved or degraded under specific pH conditions of pH 5 or higher. For example, the enteric polymer may be enteric cellulose derivatives such as hypromellose acetate succinate, hypromellose phthalate (hydroxypropylmethylcellulose phthalate), hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, and methylhydroxyethylcellulose; enteric acrylic acid copolymers such as a styrene/acrylic acid copolymer, a methyl acrylate/acrylic acid copolymer, a methyl acrylate/methacrylic acid copolymer (e.g., Acryl-EZE), a butyl acrylate/styrene/acrylic acid copolymer, and a methyl acrylate/methacrylic acid/octyl acrylate copolymer; enteric polymethacrylate copolymers such as a poly(methacrylic acid/methyl methacrylate) copolymer (e.g., Eudragit L or Eudragit S, manufactured by Evonik, Germany), and a poly(methacrylic acid/ethyl acrylate) copolymer (e.g., Eudragit L100-55); enteric maleic acid copolymers such as a vinyl acetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, and a butyl acrylate/styrene/maleic anhydride copolymer; and enteric polyvinyl derivatives such as polyvinyl alcohol phthalate, polyvinylacetal phthalate, polyvinylbutyrate phthalate, and polyvinylacetacetal phthalate.

The water-insoluble polymer refers to a pharmaceutically acceptable water-insoluble polymer which controls the release of a drug. For examle, the water-insoluble polymer may be polyvinyl acetate (e.g. Kollicoat SR30D), a water-insoluble polymethacrylate copolymer {e.g. poly(ethyl acrylate-methyl methacrylate) copolymer (such as Eudragit NE30D, a poly(ethyl acrylate-methyl methacrylate-trimethylaminoethyl methacrylate) copolymer (e.g. Eudragit RSPO), etc.}, ethylcellulose, cellulose ester, cellulose ether, cellulose acylate, cellulose diacylate, cellulose triacylate, cellulose acetate, cellulose diacetate, cellulose triacetate, etc..

The hydrophobic compound refers to a pharmaceutically acceptable water-insoluble material which controls the release of a drug. For example, the hydrophobic compound may be fatty acids and fatty acid esters such as glyceryl palmitostearate, glyceryl stearate, glyceryl behenate, cetyl palmitate, glyceryl monooleate and stearic acid; fatty acid alcohols such as cetostearyl alcohol, cetyl alcohol and stearyl alcohol; waxes such as carnauba wax, beeswax and microcrystalline wax; and inorganic materials such as talc, precipitated calcium carbonate, calcium hydrogen phosphate, zinc oxide, titanium oxide, kaolin, bentonite, montmorillonite and veegum.

The hydrophilic polymer refers to a pharmaceutically acceptable water-soluble polymer which controls the release of a drug. For example, the hydrophilic polymer may be saccharides such as dextrin, polydextrin, dextran, pectin and a pectin derivative, alginate, polygalacturonic acid, xylan, arabinoxylan, arabinogalactan, starch, hydroxypropyl starch, amylose and amylopectin; cellulose derivatives such as hypromellose, hydroxypropylcellulose, hydroxymethylcellulose, hydroxyethylcellulose, methylcellulose, and sodium carboxymethylcellulose; gums such as guar gum, locust bean gum, tragacanth, carrageenan, gum acacia, gum arabic, gellan gum and xanthan gum; proteins such as gelatin, casein and zein; polyvinyl derivatives such as polyvinyl alcohol, polyvinylpyrrolidone and polyvinylacetal diethylaminoacetate; hydrophilic polymethacrylate copolymers such as a poly(butyl methacrylate-(2-dimethylaminoethyl)methacrylate-methyl methacrylate) copolymer (e.g. Eudragit E100, manufactured by Evonik, Germany), and a poly(ethyl acrylate-methyl methacrylate-triethylaminoethyl-methacrylate chloride) copolymer (e.g. Eudragit RL and RS, manufactured by Evonik, Germany); polyethylene derivatives such as polyethylene glycol, and polyethylene oxide; and carbomer.

In addition, the composition of the present invention may be formulated with the use of pharmaceutically acceptable additives such as various additives selected from colorants and fragrances.

In the present invention, the range of the additive that can be used in the present invention is not limited to the above-mentioned additives, the additive may be appropriately selected by those skilled in the art and the composition may be formulated with the use of the above-mentioned additives in a conventional dose.

The pharmaceutical composition in accordance with the present invention may be formulated into oral dosage forms such as powders, granules, tablets, capsules, suspensions, emulsions, syrups, and aerosols, medicines for external use, suppositories or sterile injection solutions, according to a conventional known method, and may be used.

Moreover, the present invention provides a method for preventing or treating osteoporosis, the method comprising administering the compound represented by formula 1, the above-mentioned compound, or a pharmaceutically acceptable salt thereof of the present invention to a subject including a mammal. As used herein, the term "administering" means the introduction of the composition of the present invention for preventing or treating osteoporosis to a patient by any appropriate method. The composition for preventing or treating osteoporosis of the present invention may be administered via any conventional administration route as long as the composition can reach a target tissue. For example, the composition may be administered orally, intraperitoneally, intravenously, intramuscularly, subcutaneously, intradermally, intranasally, intrapulmonary, rectally, intracavitary, intraperitoneally, or intrathecally, but not limited thereto.

The present invention provides a method for preventing or treating osteoporosis, the method comprising administering a compound represented by the following formula 1 to a mammal: 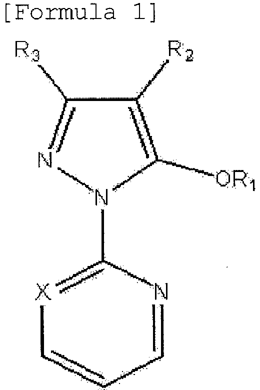 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when X represents -CH- and R₁ and R₂ each represents a hydrogen atom, R₃ represents a substituted or unsubstituted diphenylethenyl, or when X represents a nitrogen atom and R₁ and R₂ each represents a hydrogen atom, R₃ represents a nitrophenyl or a substituted or unsubstituted diphenylethenyl; and
wherein the substituent is a nitro, a hydroxyl, or a methoxyl.

The present invention provides a method for preventing or treating osteoporosis, the method comprising administering a pharmaceutically acceptable salt of a compound represented by the following formula 1 to a mammal: 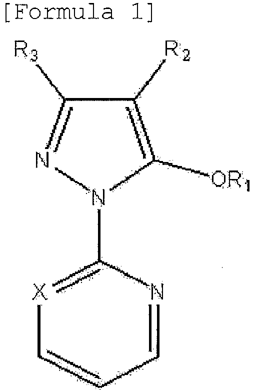 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₁ and R₂ each represents a hydrogen atom, R₃ represents a phenyl, a nitrophenyl, or a substituted or unsubstituted diphenylethenyl; and
wherein the substituent is a nitro, a hydroxyl, or a methoxyl.

The present invention provides a method for preventing or treating osteoporosis, the method comprising administering at least one compound selected from:
1-(pyridin-2-yl)-3-phenyl-4-propyl-*1H-*pyrazol-5-ol,
1-(pyridin-2-yl)-3-phenyl-4-benzyl-1*H*-pyrazol-5-ol,
1-(pyridin-2-yl)-3-phenyl-4-(4-nitrobenzyl)-*1H*-pyrazol-5-ol,
1-(pyridin-2-yl)-3-phenyl-4-propyl-1*H*-pyrazol-5-ol hydrochloride,
1-(pyridin-2-yl)-3-phenyl-4-propyl-*1H*-5-(isopropyloxycarbonyloxymethyloxy)pyrazole,
1-(pyridin-2-yl)-3-[(3-methoxy-4-hydroxyphenyl)-E-ethenyl]-4-benzyl-1*H*-pyrazol-5-ol hydrochloride,
1-(pyridin-2-yl)-3-[(3-methoxy-4-hydroxyphenyl)-E-ethenyl]-4-benzyl-1*H*-pyrazol-5-ol,
3-(4-nitrophenyl)-3-[2-(pyrimidin-2-yl)hydrazono]-propionic acid ethyl ester,
1-(pymiridin-2-yl)-3-(4-nitrophenyl)-5-*1H-*pyrazol-5-ol,
1-(pyridin-2-yl)-3-(1,2-diphenyl-*E*-ethenyl)-*1H*-pyrazol-5-ol,
1-(pyridin-2-yl)-3-phenyl-*1H*-pyrazol-5-ol hydrochloride,
3-(4-methoxyphenylamino)-1*H*-pyrazol-5-ol, and
4-hexylidene-3-phenyl-1-(pyridin-2-yl)-*1H*-pyrazol-5-ol;
or a pharmaceutically acceptable salt thereof to a mammal.

The composition for preventing or treating osteoporosis in accordance with the present invention may be administered once a day or may be administered at regular time intervals twice or more a day.

The dosage of the compound represented by formula 1, the above-mentioned compound, or a pharmaceutically acceptable salt thereof of the present invention varies depending on body weight, age, gender, and health state of the patient, diet, administration timing, administration route, excretion rate, severity of the disease, etc.. Suitable dosages may be 0.1 to 100 mg/kg/day, more preferably 10 to 40 mg/kg/day, but may vary depending on the patient's severity, age, sex, etc..

Moreover, the present invention provides a method for inhibiting the generation of reactive oxygen species, the method comprising administering the compound represented by formula 1, the above-mentioned compound, or a pharmaceutically acceptable salt thereof to a subject including a mammal.

The present invention provides a method for inhibiting the production of osteoclasts, the method comprising administering the compound represented by formula 1, the above-mentioned compound, or a pharmaceutically acceptable salt thereof to a subject including a mammal.

The present invention also provides a use of the compound represented by formula 1, the above-mentioned compound, or a pharmaceutically acceptable salt thereof for preparing a pharmaceutical preparation for the prevention or treatment of osteoporosis.

Moreover, the present invention provides a health food comprising the compound represented by formula 1, the above-mentioned compound, or a pharmaceutically acceptable salt thereof. The health food may preferably be a health food for strengthening bone.

The present invention provides a reactive oxygen species inhibitor for inhibiting the generation of reactive oxygen species, comprising the compound represented by formula 1, the above-mentioned compound, or a pharmaceutically acceptable salt thereof.

### [Advantageous Effects]

The compounds of the present invention have excellent inhibitory activity on the generation of reactive oxygen species and can be used for the treatment or prevention of osteoporosis without any special side effects of conventional therapeutic agents.

### [Description of Drawings]

FIGS. 1 to 6 are views showing the inhibitory effect of compounds according to the present invention on osteoclast differentiation.

FIGS. 7 and 8 are views showing the inhibitory effect of compounds according to the present invention on lipopolysaccharide (LPS)-induced osteolysis.

FIGS. 9 to 13 are views showing the inhibitory effect of compounds according to the present invention on bone loss due to ovariectomy.

### [Mode for Invention]

Hereinafter, the present invention will be described in more detail with reference to the following Examples and Experimental Examples. However, it should be understood that the following examples and Experimental Examples are provided only for illustrating the present invention and should not be construed as limiting the scope and spirit of the present invention.

Reagents and solvents mentioned below were those available from Sigma-Aldrich Korea, Alfa Aesar, or Tokyo Chemical Industry (TCI). Moreover, ¹H-NMR and ¹³C-NMR spectra were obtained with a JEOL Eclipse FT 300 MHz Spectrometer and mass spectra was obtained with a JEOL MStation JMS 700 mass Spectrometer.

### Example 1: Synthesis of 1-(pyridin-2-yl)-3-phenyl-4-propyl-1H-pyrazol-5-ol

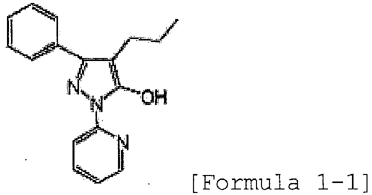

2-propyl-3-oxo-3-phenylpropionic acid ethyl ester (2.52 g, 10.7 mmol) and ethanol (10 mL) were placed in a round-bottom flask, and 2-hydrazinopyridine (1.29 g, 11.8 mmol) diluted with ethanol (10 mL) was slowly added dropwise at 0 °C. The resulting solution was heated under reflux at 100 °C for 3 days. The solvent was removed by distillation under reduced pressure, and the resulting solid was washed with hexane and ethyl acetate and dried under vacuum to give the target compound in 82% yield.

¹H NMR(300 MHz, CDCl₃) 5 12.50 (1H, s), 8.27-8.25 (1H, m), 8 .01 (1H, d, J = 8.5 Hz), 7.81 (1H, m), 7.69 (2H, m), 7.48-7.34 (3H, m), 7.14-7.10 (1H, m), 2.54 (2H, d, J= 7.5 Hz), 1.64 (2H, m), 0.93 (3H, t, J = 7.3 Hz);

EIMS (70 eV) m/z (rel intensity) 279 (M +, 37), 250 (100).

### Example 2: Synthesis of 1-(pyridin-2-yl)-3-phenyl-4-benzyl-1H-pyrazol-5-ol

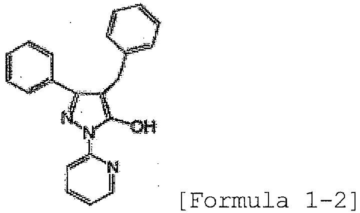

2-benzyl-3-oxo-3-phenyl-propionic acid ethyl ester (530 mg, 1.87 mmol) and ethanol (7 mL) were placed in a round-bottom flask, and 2-hydrazinopyridine (204 mg, 1.87 mmol) diluted with ethanol (3 mL) was slowly added dropwise at 0 °C. The resulting solution was heated under reflux at 100 °C for 2 days. The solvent was removed by distillation under reduced pressure, and the resulting solid was washed with hexane and ethyl acetate and dried under vacuum to give the target compound in 92% yield.
¹H NMR(300 MHz, CDCl₃) δ 12.66 (1H, s), 8.27-8.25 (1H, m), 8.01 (1H, d, J = 8.5 Hz), 7.81 (1H, m), 7.63 (2H, m), 7.35-7.12 (9H, m), 3.93 (2H, s);
EIMS (70 eV) m/z (rel intensity) 327 (M+, 100), 250 (75), 222 (32), 206 (20), 195 (15).

### Example 3: Synthesis of 1-(pyridin-2-yl)-3-phenyl-4-(4-nitrobenzyl)-1H-pyrazol-5-ol

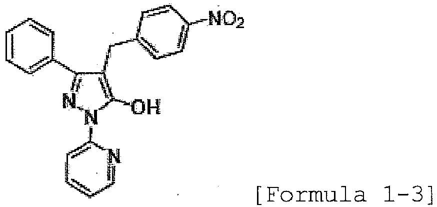

2-(4-nitrobenzyl)-3-oxo-3-phenyl-propionic acid ethyl ester (142 mg, 0.43 mmol) and ethanol (5 mL) were placed in a microwave reactor vial, and 2-hydrazinopyridine (47 mg, 0.43 mmol) diluted with ethanol (3 mL) was slowly added dropwise at 0 °C. The resulting solution was heated in a microwave reactor at 120 °C for 2 days to give the target compound in 29% yield.

¹H NMR(300 MHz, CDCl3) δ 12.82 (1H, s), 8.30 (1H, d, J = 4.4 Hz), 8.12-7.48 (4H, m), 7.71-7.17 (8H, m), 3.01 (2H, s); EIMS (70 eV) m /z (rel intensity) 372 (M+, 0.01), 250 (15), 222 (12), 189 (11), 147 (20), 121 (78), 105 (89).

### Example 4: Synthesis of 1-(pyridin-2-yl)-3-phenyl-4-propyl-1H-pyrazol-5-ol·HCl

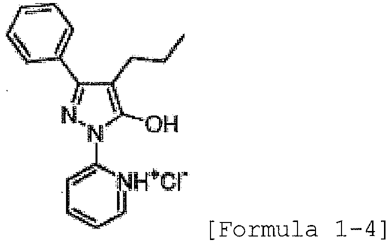

1-(pyridin-2-yl)-3-phenyl-4-propyl-*1H*-pyrazol-5-ol (280 mg) prepared in Example 1 was dissolved in ethyl ether (4 mL) in a round-bottom flask, and ethyl ester (0.55 mL) in which 2 M HCl was dissolved was slowly added dropwise at 0 °C. The solvent was removed by vacuum filtration, and the resulting solid was washed with hexane and ethyl acetate and dried under vacuum to give the target compound (270 mg).
¹H NMR (300 MHz, DMSO-d₆) δ 8.44 (1H, d, J = 4.2 Hz), 8.08-8.03 (2H, m), 7.66-7.64 (2H, m), 7.48-7.42 (3H, m), 7.34 -7.30 (1H, m), 2.49 (2H, brs), 2.43 (2H, t, J = 7.5 Hz), 1.48 (2H, m), 0.48 (3H, t, J = 7.3 Hz).

### Example 5: Synthesis of 1-(pyridin-2-yl)-3-phenyl-4-propyl-1H-5-(isopropyloxycarbonyloxymethyloxy)pyrazole (18-306)

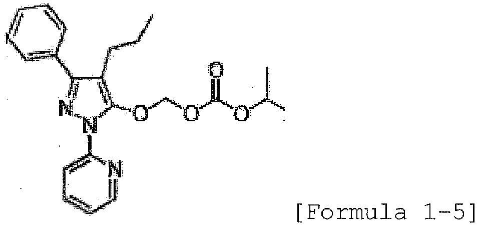

1-(pyridin-2-yl)-3-phenyl-4-propyl-*1H*-pyrazol-5-ol (438 mg) prepared in Example 1, K₂CO₃ (650 mg), and Bu₄NHSO₄ (532 mg) were added to a mixed solvent of water (8 mL) and dichloromethane (8 mL), and a solution in which isopropyloxycarbonyloxymethyliodide (497 mg) was dissolved in dichloromethane (2 mL) was added thereto. Then, the resulting solution was vigorously stirred overnight such that the compounds were reacted. The organic layer obtained by extracting the resulting solution with dichloromethane was washed with water and brine and concentrated. The resulting residue was purified by column chromatography (developing solvent: hexane/ethyl acetate (EtOAc) = 10/1) to give the target compound (361 mg, 58% yield).

¹H NMR(300 MHz, CDCl₃) δ 8.48-8.46 (1 H, m), 7.89 (1H, d, J = 8.2 Hz), 7.80 (1H, m), 7.78-7.68 (2H, m), 7.57-7.34 (3H, m), 7.23-7.1 6 (1H, m), 5.84 (2H, s), 4.86 (1H, m), 2.55 (2H, d, J = 7.7 Hz), 1.58 (2H, m), 1.25 (6H, d, J = 6.3 Hz), 0.91 (3H, t, J = 7.5 Hz)

¹³C NMR(75 MHz, CDCl3) δ 153.6, 152.0, 150.8, 149.3, 147.9, 138.3, 134.0, 128.4, 128.0, 127.6, 121.3, 115.9, 110.0, 92.6, 72.7, 24.7, 22.9, 21.6, 14.1.

### Example 6: Synthesis of 1-(pyridin-2-yl)-3-[(3-methoxy-4-hydroxyphenyl)-E-ethenyl]-4-benzyl-1H-pyrazol-5-ol·HCl

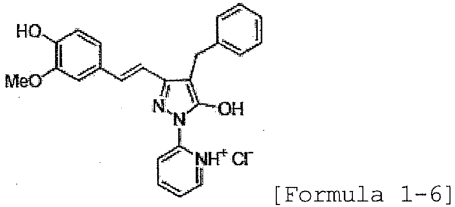

2-benzyl-3-oxo-3-[(3-methoxy-4-hydroxyphenyl)-E-ethenyl]-propionic acid ethyl ester (3.55 g, 10 mmol) and acetic acid (10 mL) were placed in a round-bottom flask, and 2-hydrazinopyridine (1.1 g, 10.1 mmol) diluted with acetic acid (3 mL) was slowly added dropwise at 0 °C. The resulting solution was heated under reflux at 100 °C for 2 days. The solvent was removed by distillation under reduced pressure, and the residue was purified by column chromatography (developing solvent: hexane/ethyl acetate (EtOAc) = 15/1) to give 1-(pyridin-2-yl)-3-[(3-methoxy-4-hydroxyphenyl)-E-ethenyl]-4-benzyl-1*H*-pyrazol-5-ol (1.32g).

The obtained 1-(pyridin-2-yl)-3-[(3-methoxy-4-hydroxyphenyl)-*E*-ethenyl]-4-benzyl-*1H*-pyrazol-5-ol (1.10g) was dissolved in ethyl ether (15 mL) in a round-bottom flask, and ethyl ether (1.5 mL) in which 2 M HCl was dissolved was slowly added dropwise at 0 °C. The resulting solution was stirred at 40 °C for 24 hours, the solvent was removed by vacuum filtration, and then the resulting solid was washed with hexane and ethyl acetate and dried under vacuum to give the target compound (1.1 g).

¹H NMR (300 MHz, DMSO-d₆) δ 8.47-8.45 (1H, m), 8.30 (1H, d, J = 8.4 Hz), 7.98-7.93 (2H, m), 7.32-6.83 (10H, m), 3.82 (3H, s), 2.72 (2H, s).

### Example 7: Synthesis of 3-(4-nitrophenyl)-3-[(pyrimidin-2-yl)hydrazono]-propionic acid ethyl ester

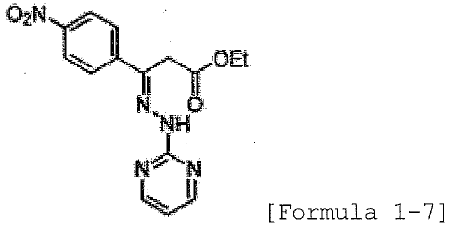

Ethyl-4-nitrobenzoylacetate (356 mg) and 2-hydrazinopyrimidine (182 mg) were dissolved in ethanol (15 mL) in a round-bottom flask. The resulting mixture was heated under reflux at 100 °C for 3 days. The organic layer obtained by extracting the resulting solution with ethyl acetate was washed with water and brine and concentrated. The resulting residue was purified by column chromatography (developing solvent: hexane/ethyl acetate (EtOAc) = 1/1) to give the target compound (118 mg, 24% yield).

¹H NMR(300 MHz, CDCl₃) δ 9.79 (1H, s), 8.56 (2H, d, *J* = 4.8 Hz), 8.26 (2H, d, J = 7.2 Hz), 8.09 (2H, d, J = 7.0 Hz), 6.89 (1H, t, J= 4.9 H z), 4.28 (2H, q, J =7.2 Hz), 3.89 (2H, s), 1.28 (3H, t, J = 7.2 Hz).

### Example 8: Synthesis of 1-(pyrimidin-2-yl)-3-(4-nitrophenyl)-1H-pyrazol-5-ol

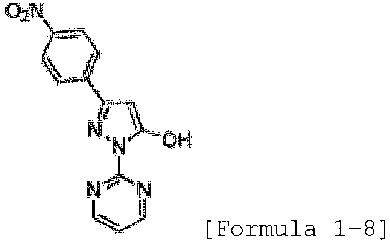

3-(4-nitrophenyl)-3-[2-(pyrimidin-2-yl)hydrazono]-propionic acid ethyl ester (100 mg) prepared in Example 7 was dissolved in acetic acid (15 mL) in a round-bottom flask. The resulting mixture was heated under reflux at 150 °C for 2 days. Ethyl acetate was added to the concentrate obtained by removing the solvent, and the organic layer was washed with dilute NaHCO₃ aqueous solution and water and concentrated. The resulting residue was purified by column chromatography (developing solvent: hexane/ethyl acetate (EtOAc) = 1/1) to give the target compound (61 mg, 72% yield).

¹H NMR(300 MHz, CDCl₃) δ 11.9 (1H, s), 8.83 (2H, d, J = 4.8 Hz), 8.27 (2H, d, J = 7.2 Hz), 8.08 (2H, d, J = 7.0 Hz), 7.30 (1H, t, J = 4.7 Hz), 6.09 (1H, s).

### Example 9: Synthesis of 1-(pyridin-2-yl)-3-[(1,2-diphenyl-E-ethenyl]-1H-pyrazol-5-ol

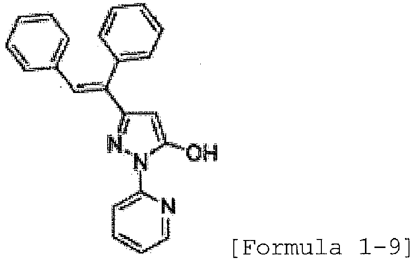

4,5-diphenyl-3-oxo-4-pentenoic acid ethyl ester (294 mg) and 2-hydrazinopyridine (120 mg) were dissolved in acetic acid (5 mL) in a round-bottom flask. The resulting mixture was heated under reflux at about 150 °C for 5 hours. After removing the solvent, the organic layer obtained by extracting the resulting solution with ethyl acetate was washed with water and brine and concentrated. The resulting residue was purified by column chromatography (developing solvent: hexane/ethyl acetate (EtOAc) = 10/1) to give the target compound (130 mg, 38% yield).
¹H NMR(300 MHz, CDCl₃) δ 12.7 (1H, s), 8.26-7.81 (3H, m), 8.43-7.03 (12H, m), 5.46 (1H, s);
EIMS (70 eV) m/z (rel intensity) 339 (M+, 100), 310 (6), 262 (23), 217 (21), 202 (24), 178 (18), 121 (48).

### Example 10: Synthesis of 1-(pyridin-2-yl)-3-phenyl-1H-pyrazol-5-ol·HCl

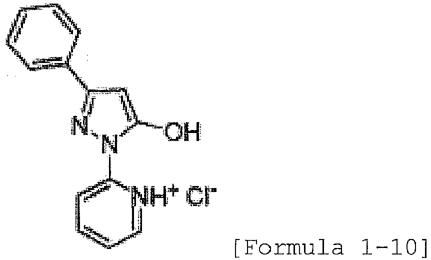

Ethyl benzoylacetate (1.92 g, 9.99 mmol) and ethanol (10 mL) were placed in a round-bottom flask, and 2-hydrazinopyridine (1.1 g, 10.0 mmol) diluted with ethanol (10 mL) was slowly added dropwise at 0 °C. The resulting solution was heated under reflux at 100 °C for 8 hours. The solvent was removed by distillation under reduced pressure, and the resulting solid was washed with hexane and ethyl acetate and dried under vacuum to give 1-(pyridin-2-yl)-3-phenyl-*1H-*pyrazol-5-ol in 87% yield.

Ethyl ether (4 mL) was added to the prepared 1-(pyridin-2-yl)-3-phenyl-*1H*-pyrazol-5-ol (237 mg) in a round-bottom flask, and ethyl ether (0.55 mL) in which 2 M HCl was dissolved was slowly added dropwise at 0 °C. The solvent was removed by vacuum filtration, and the resulting solid was washed with hexane and ethyl acetate and dried under vacuum to give the target compound in 87% yield.

¹H NMR (300 MHz, DMSO-d₆) δ 8.48-8.46 (m, 1H), 8.08 (t, 1H, J = 8.3 Hz), 7.96 (d, 1H, J = 8.4 Hz), 7.87 (d, 2 H, *J* = 8.3 Hz), 7.46-7.35 (m, 4H), 6.64 (br, 4H), 6.14 (s, 1H).

### Example 11: Synthesis of 4-hexylidene-3-phenyl-1-(pyridin-2-yl)-1H-pyrazol-5-ol

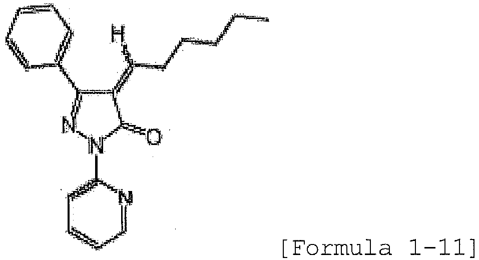

Ethyl benzoylacetate (1.92 g, 9.99 mmol) and ethanol (10 mL) were placed in a round-bottom flask, and 2-hydrazinopyridine (1.1 g, 10.0 mmol) diluted with ethanol (10 mL) was slowly added dropwise at 0 °C. The resulting solution was heated under reflux at 100 °C for 8 hours. The solvent was removed by distillation under reduced pressure, and the resulting solid was washed with hexane and ethyl acetate and dried under vacuum to give 1-(pyridin-2-yl)-3-phenyl-*1H-*pyrazol-5-ol.

The prepared 1-(pyridin-2-yl)-3-phenyl-*1H*-pyrazol-5-ol (2.37 g) was placed in a round-bottom flask, and hexanal (50 mL) was added thereto. The resulting solution was heated under reflux at 150 °C for 12 hours, the solvent was removed by distillation under reduced pressure, and the resulting reddish brown solid was recrystallized in ethanol to give the target compound in 76% yield.
¹H NMR (300MHz, CDCl3) δ 8.26-7.80 (3H, m), 7.43-7.03 (6H, m), 6.18 (1H, br s), 2.01 (2H, m), 1.34-1.25 (6H, m), 0.97 (3H, t, J = 7.6Hz).

### Example 12: Synthesis of 3-(4-methoxyphenylamino)-1H-pyrazol-5-ol

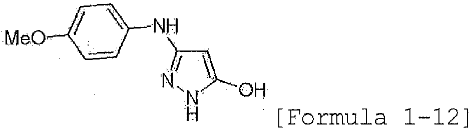

3-amino-5-hydroxypyrazole (9.91 g, 100 mmol), *p*-anisole (15.40 g, 125 mmol), and acetic acid (200 mL) were placed in a round-bottom flask and heated under reflux at 140 °C for 4 hours. The solvent was removed by distillation under reduced pressure, and ice water (70 mL) was added thereto. The resulting solid was filtered and recrystallized in ethanol to give the target compound in 23% yield.

¹H NMR(300 MHz, DMSO-d₆) δ 10.23 (1H, s), 8.49 (1H, s), 7.40 (2H, d, J= 9.0 Hz), 6.82 (2H, d, J = 9.0 Hz), 3.67 (3H, s), 3.31 (1H, s);

EIMS (70 eV) m/z (rel intensity) 205 (M+, 100 ), 190 (53), 174 (42), 148 (50), 121 (50), 104 (6), 92 (9), 77 (15).

### Example 13: Synthesis of 1-(pyridin-2-yl)-3-[(3-methoxy-4-hydroxyphenyl)-E-ethenyl]-4-benzyl-1H-pyrazol-5-ol

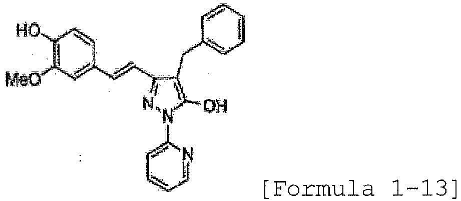

2-benzyl-3-oxo-3-[(3-methoxy-4-hydroxyphenyl)-E-ethenyl]-propionic acid ethyl ester (3.55g, 10 mmol) and acetic acid (10 mL) were placed in a round-bottom flask, and 2-hydrazinopyridine (1.1 g, 10.1 mmol) diluted with acetic acid (3 mL) was slowly added dropwise at 0 °C. The resulting solution was heated under reflux at 100 °C for 2 days. The solvent was removed by distillation under reduced pressure, and the residue was purified by column chromatography (developing solvent: hexane/ethyl acetate (EtOAc) = 15/1) to give 1-(pyridin-2-yl)-3-[(3-methoxy-4-hydroxyphenyl)-E-ethenyl]-4-benzyl-1*H*-pyrazol-5-ol (1.32g).

¹H NMR (300 MH z, CDCl₃) δ 8.26 (1H, m), 7.98 (1H, m), 7.83 (1H, m), 7.37-7.13 (8H, m), 7.11 (1H, d, J = 12.1 Hz), 6.91(1H, m), 6.81 (1H, d, J= 12.0 Hz), 3.93 (2H, s), 3.90 (3H, s).

### Comparative Example 1: Synthesis of 3-phenyl-1-(pyridin-2-yl)-1H-pyrazol-5-ol

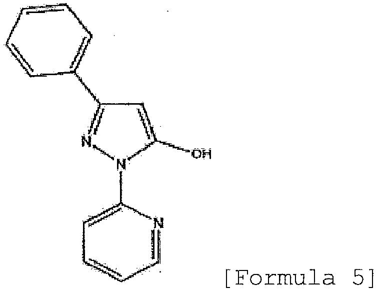

3-phenyl-1-(pyridin-2-yl)-1H-pyrazol-5-ol was synthesized by the method disclosed in Korean Patent No. KR 0942382.

Ethyl benzoylacetate (1 equivalent) and ethanol (4 mL) were placed in a round-bottom flask, and 2-hydrazinopyridine (1.1 equivalents) diluted with ethanol (3 mL) was slowly added dropwise at 0 °C. The resulting solution was heated under reflux for 20 minutes. The solvent was removed by distillation under reduced pressure, and the resulting solid was washed with hexane and ethyl acetate and dried under vacuum to give the target compound in 87% yield.

### Experimental Example 1: Determination of Inhibitory Effect of Compounds on Differentiation into Osteoclasts

Bone marrow cells were harvested from 4 to 5-week old male mice (C57BL/6J). Specifically, mice were sacrificed by cervical dislocation, and femur and tibia were extracted while removing muscles adhered around the bone with scissors and soaked in phosphate buffered saline (PBS). A 1 mL syringe filled with α-minimum essential medium (α-MEM) was put into one end of the femur and tibia from which bone marrow cells were then harvested.

The bone marrow cells were cultured to obtain macrophages. Specifically, the obtained bone marrow cells were placed in a 50 mL tube and centrifuged at 1500 rpm for 5 minutes. Then, the supernatant was removed, and a 3:1 mixture of a Gey's solution and PBS was added and maintained at room temperature for about 2 to 3 minutes. After another centrifugation (1500 rpm, 5 minutes), the supernatant was removed, α-MEM was added, followed by stirring, and then the cells were cultured in a 10 cm cell culture dish at 37 °C for 24 hours. After the third centrifugation (1500 rpm, 5 minutes), the supernatant was removed, a culture medium and a macrophage differentiation factor rhM-CSF (30 ng/mL) were added, and then the cells were cultured in a 10 cm cell culture dish at 37 °C for 3 days. After 3 days, macrophages adhered to the dish were scraped and collected in a tube, followed by centrifugation (1500 rpm, 5 minutes).

The macrophages were cultured to induce cellular differentiation into osteoclasts. Specifically, the obtained macrophages were aliquoted at a density of 2x10⁴ cells/well in a 48-well cell culture dish and cultured for 24 hours. rhM-CSF (30 ng/mL) and an osteoclast differentiation factor RANKL (200 ng/mL) were added to the culture medium and cultured to induce cellular differentiation into osteoclasts. At this time, each of the compounds synthesized in Examples 2, 3, 7, 8, 9 and 11 was added to the culture medium at various concentrations, and the compound of Comparative Example 1 was added at the same concentrations as the compounds of the above Examples in a control group for comparison.

After 24 hours, the culture medium in the 48-well cell culture dish was removed and replaced with a fresh medium and cultured at 37 °C with replacement of the culture medium every two days.

After further culturing for 5 days from the day on which each of the compounds synthesized in the Examples of the present invention was added to the medium, the medium to which each of the compounds synthesized in the Examples of the present invention was added and the medium of the control group were fixed in a 3.7% formalin solution, stained with tartrate resistant acid phosphatase (TRAP), and examined under a light microscope. Specifically, the TRAP staining was carried out as follows: the cells were fixed in 3.7% formaldehyde at room temperature for 15 minutes and washed twice with distilled water, and then a staining solution prepared by mixing acetate, Fast Gargnet GBC base, naphthol AS-B1 phosphoric acid, sodium nitride, and tartrate in the ratio described in the instructions attached to an Acid Phosphatase, Leukocyte (TRAP) Kit™ (Sigma Co.) was added at a dose of 200 µL/well, followed by reaction at 37 °C for 20 minutes.

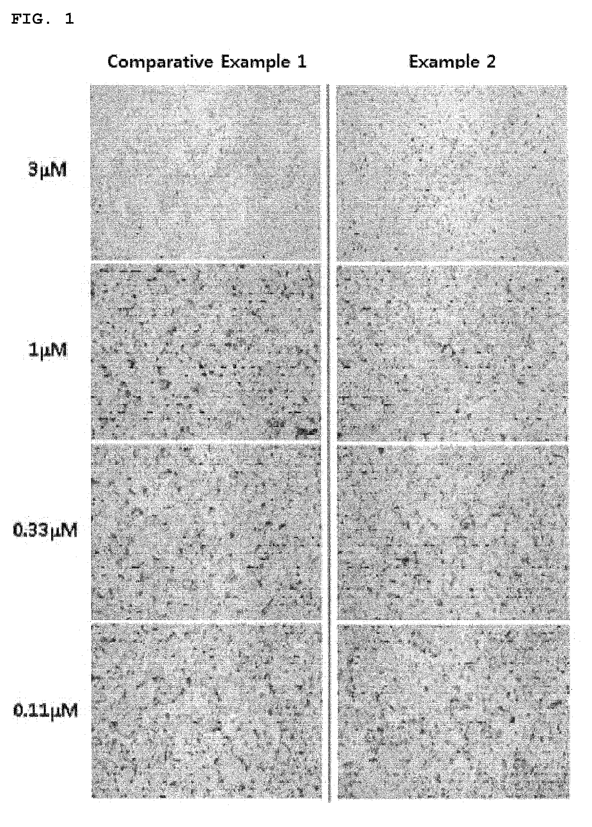

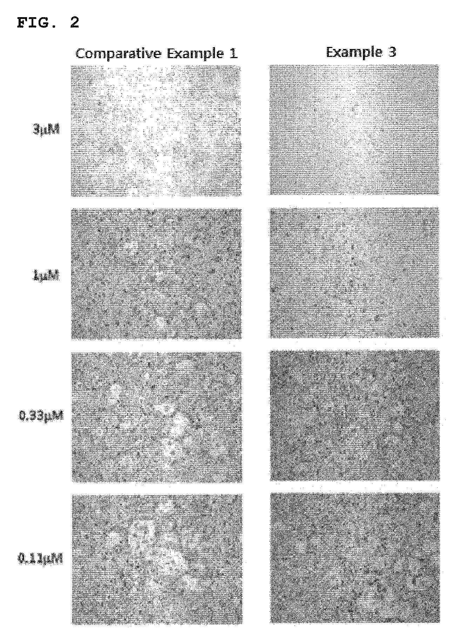

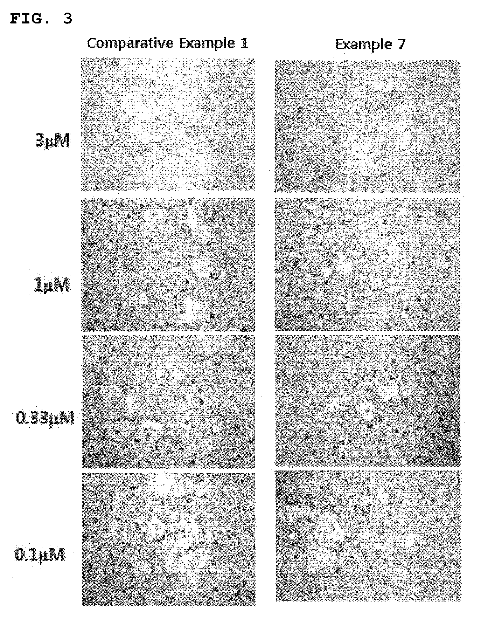

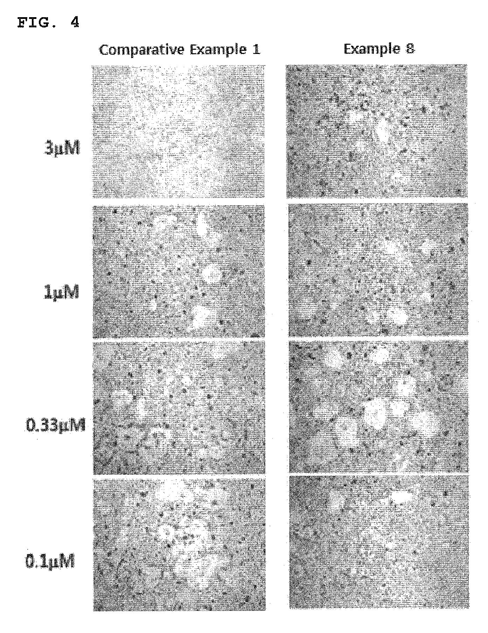

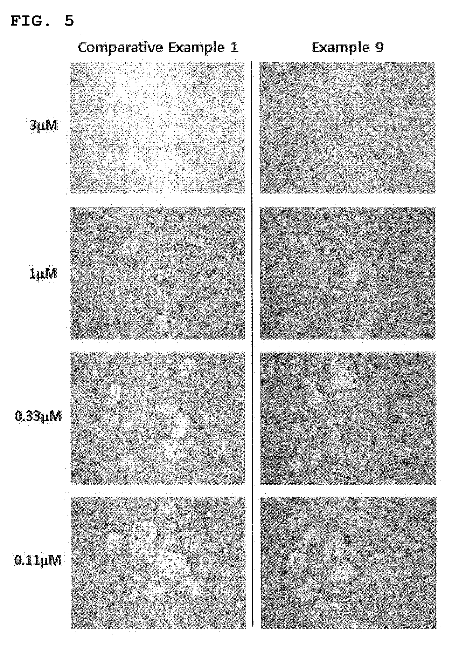

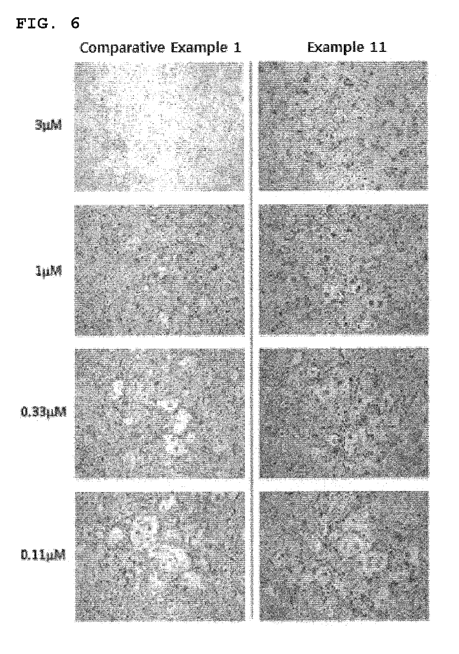

The experimental results are shown in FIGS. 1 to 6. As shown in FIGS. 1 to 6, it can be seen that in the control group added the compound of Comparative Example 1, more macrophages differentiated into osteoclasts, while the macrophages in the medium added the compounds synthesized in the Examples of the present invention did not exhibit normal differentiation into osteoclasts or exhibited less differentiation into osteoclasts. Thus, it can be seen that the compounds of the present invention can inhibit the differentiation of macrophages into osteoclasts.

Moreover, it can be seen that the differentiation into osteoclasts was inhibited when the concentration of the compounds synthesized in the Examples of the present invention increased to 0.1 µM or 0.11 µM, 0.33 µM, 1 µM, and 3 µM. Thus, it can be seen that the compounds of the present invention can inhibit the differentiation of macrophages into osteoclasts in a dose-dependent fashion.

### Experimental Example 2: Determination of Inhibitory Effect of Compounds on Osteoclastic Activity due to Inflammation Inducing Factor

In order to examine the inhibitory effects of the compounds on the osteoclastic activity induced by lipopolysaccharide (LPS) in bone loss mouse models, 6-week old C57BL/6J male mice were divided into control groups 1 to 3 and experimental groups 1 to 3, each containing 3 mice.

Each mouse was anesthetized and the skin in the middle of the skull was incised about 1 cm and packed with a collagen sponge (0.5 mm x 0.5 mm).

PBS and DMSO were respectively injected into the sponges of control groups 1 and 2, and LPS (12.5mg/Kg) was injected into the sponges of control group 3. LPS (12.5mg/Kg) and the compound (20mg/Kg) of Example 4 were injected into the sponges of experimental group 1, LPS (12.5mg/Kg) and the compound (20mg/Kg) of Example 5 were injected into the sponges of experimental group 2, and LPS (12.5mg/Kg) and the compound (20mg/Kg) of Example 6 were injected into the sponges of experimental group 3.

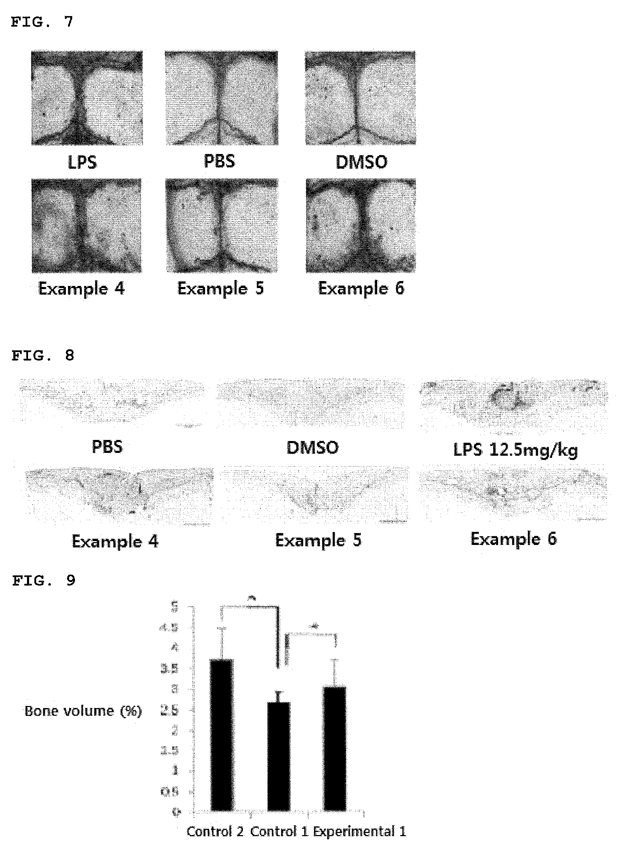

Then, the incised skin was sutured, and skulls were removed from the mice after 5 days. The removed skulls were placed in PBS added 4% formaldehyde and fixed for 24 hours. Then, the skulls were decalcified in PBS added 0.5 M EDTA and subjected to TRAP staining to observe the surface of the skulls under a microscope, and the results are shown in FIG. 7.

The TRAP staining was carried out by placing the skulls in a staining solution prepared by mixing acetate, Fast Gargnet GBC base, naphthol AS-B1 phosphoric acid, sodium nitride, and tartrate in the ratio described in the instructions attached to an Acid Phosphatase, Leukocyte (TRAP) Kit™ (Sigma Co.) at a dose of 200 µL/well, followed by reaction at 37 °C for 20 minutes.

Moreover, the decalcified skulls were placed in paraffin to form blocks, and the blocks were cut at a thickness of 0.4 µm using a microtome and hydrated after removing the paraffin. The cut blocks were stained with hematoxylin and TRAP and observed under a microscope, and the results are shown in FIG. 8.

As can be seen from FIGS. 7 and 8, the degree of TRAP staining in experimental groups 1 to 3 was lower than that in control group 3 treated with LPS alone. Thus, it can be seen that the compounds of the present invention can inhibit the formation of osteoclasts by reducing the degree of inflammation due to LPS, thereby effectively inhibiting bone destruction.

### Experimental Example 3: Determination of Inhibitory Effect of Compounds on Bone Loss due to Ovariectomy

In order to examine the inhibitory effects of the compounds on the bone loss induced by ovariectomy in ovariectomized mouse models, 8-week old female mice were divided into control groups 1 and 2 and experimental group 1, each containing 5 mice.

The mice in control group 1 and experimental group 1 were subjected to ovariectomy via laparotomy, and the mice in control group 2 were subjected to laparotomy in the same manner as in control group 1 and experimental group 1 without ovariectomy and served as a sham control.

The compound of Example 13 was intraperitoneally administered to the mice of experimental group 1 for 3 weeks at intervals of 2 days after 7 days of recovery from the surgery. After verification of excision of ovary of mice, both tibiae were harvested from the mice of experimental group 1 and control groups 1 and 2, whose cross-sections were consecutively imaged using µ-CT (Skyscan 1076, SKYS CAN N. V.) and V-works program (Cybermed) and reconstructed into 3-dimensional images. Then, the bone volume, bone density, and the number of trabecular bones were quantified using an image analysis program, and the results are shown in FIGS. 9 to 11.

The experimental results and statistical analysis using ANOVA (one way analysis of variance) if verify the Newman kulseu (Newman-Keuls test) significance is verified using the p < 0.1 (*) or p < 0.15 (**) level or less, a significance test was performed.

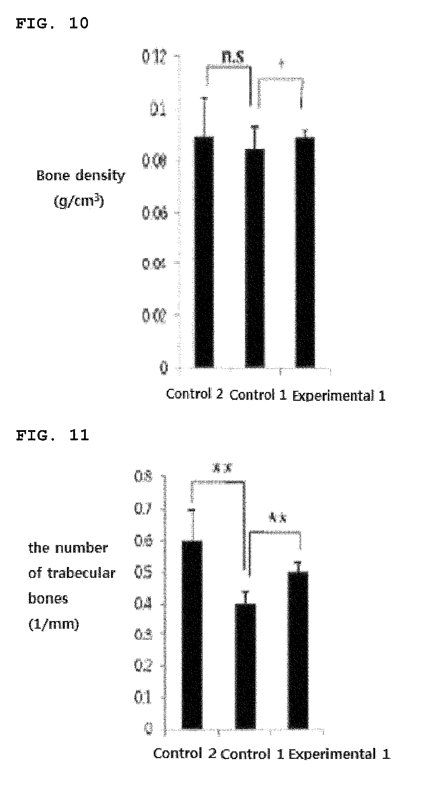

In FIG. 9, the bone volume is expressed as a percentage (BV/TV %) of the bone volume (BV) of the harvested tibia to the total bone volume (TV), and in FIG. 11, the number of trabecular bones is expressed as the number of trabecular bones) per 1 millimeter(mm).

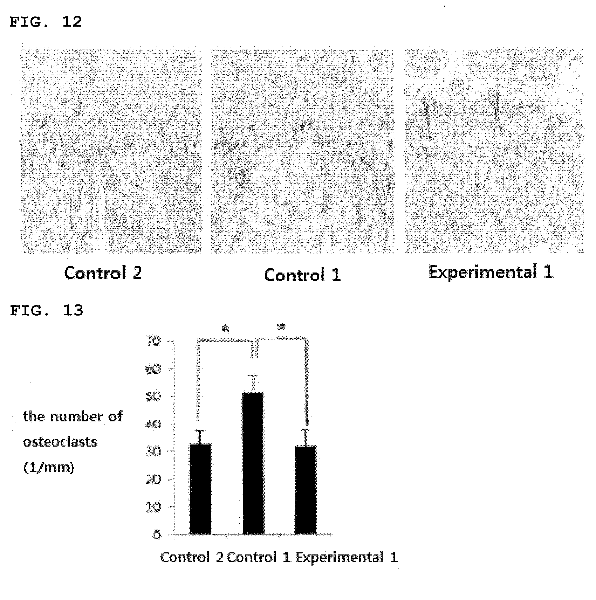

Moreover, the harvested tibiae were placed in PBS added formaldehyde and fixed for 24 hours and then decalcified in PBS added 0.5 M EDTA. Then, the tibiae were placed in paraffin to form blocks, and the blocks were cut at a thickness of 0.4 µm using a microtome and hydrated after removing the paraffin. The cut blocks were stained with hematoxylin and TRAP and observed under a microscope, and the results are shown in FIG. 12. Moreover, the number of osteoclasts was analyzed under a microscope using the Osteomeasure software program (Osteometrics Inc., USA), and the results are shown in FIG. 13, in which the quantification of osteoclasts is expressed as the number of osteoclasts per bone surface 1_millimeter (mm).

The experimental results and statistical analysis using ANOVA (one way analysis of variance) if verify the Newman kulseu (Newman-Keuls test) significance is verified using the p < 0.1 (*) or level or less, a significance test was performed.

As can be seen from FIGS. 9 to 11, in the case of experimental group 1 injected the compound of Example 13, the reduction in bone volume of the tibia was less than that in control group 1 at the same ovariectomy, and it exhibited a higher bone density and a higher number of trabecular bones. Thus, it can be found that the compounds of the present invention can effectively treat osteoporosis by inhibiting bone loss.

Moreover, as can be seen from FIG. 12, the degree of staining with TRAP in experimental group 1 was lower than that in control group 1, resulting in less formation of osteoclasts. Furthermore, as can be seen from FIG. 13, in experimental group 1, the number of osteoclasts was similar to that in control group 2 without ovariectomy, and the formation of osteoclasts was inhibited about 60% compared to the control group 1 with ovariectomy. Thus, it can be seen that the compounds of the present invention can effectively prevent and treat osteoporosis by inhibiting the formation of osteoclasts.

### [Industrial Applicability]

The compounds of the present invention have excellent inhibitory activity on the generation of reactive oxygen species and can be used for the treatment or prevention of osteoporosis without any special side effects of conventional therapeutic agents.

## Claims

1. A compound represented by the following formula 1: 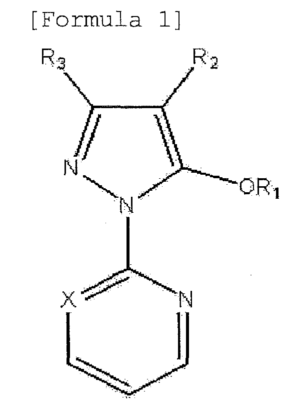 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when X represents -CH- and R₁ and R₂ each represents a hydrogen atom, R₃ represents a substituted or unsubstituted diphenylethenyl, or when X represents a nitrogen atom and R₁ and R₂ each represents a hydrogen atom, R₃ represents a nitrophenyl or a substituted or unsubstituted diphenylethenyl; and
wherein the substituent is a nitro, a hydroxyl, or a methoxyl.

2. A pharmaceutically acceptable salt of a compound represented by the following formula 1: 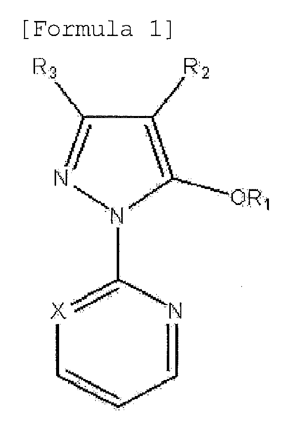 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₁ and R₂ each represents a hydrogen atom, R₃ represents a phenyl, a nitrophenyl, or a substituted or unsubstituted diphenylethenyl; and
wherein the substituent is a nitro, a hydroxyl, or a methoxyl.

3. The pharmaceutically acceptable salt of claim 2, wherein the pharmaceutically acceptable salt is a hydrochloric acid salt.

4. A compound selected from:
1-(pyridin-2-yl)-3-phenyl-4-propyl-1*H*-pyrazol-5-ol,
1-(pyridin-2-yl)-3-phenyl-4-benzyl-1*H*-pyrazol-5-ol,
1-(pyridin-2-yl)-3-phenyl-4-(4-nitrobenzyl)-1*H*-pyrazol-5-ol,
1-(pyridin-2-yl)-3-phenyl-4-propyl-1*H*-pyrazol-5-ol hydrochloride,
1-(pyridin-2-yl)-3-phenyl-4-propyl-1*H*-5-(isopropyloxycarbonyloxymethyloxy)pyrazole,
1-(pyridin-2-yl)-3-[(3-methoxy-4-hydroxyphenyl)-E-ethenyl]-4-benzyl-1*H*-pyrazol-5-ol hydrochloride,
3-(4-nitrophenyl)-3-[2-(pyrimidin-2-yl)hydrazono]-propionic acid ethyl ester,
1-(pyrimidin-2-yl)-3-(4-nitrophenyl)-1*H*-pyrazol-5-ol, 1-(pyridin-2-yl)-3-(1,2-diphenyl-E-ethenyl)-1*H*-pyrazol-5-ol,
1-(pyridin-2-yl)-3-[(3-methoxy-4-hydroxyphenyl)-E-ethenyl]-4-benzyl-*1H*-pyrazol-5-ol,
1-(pyridin-2-yl)-3-phenyl-*1H*-pyrazol-5-ol hydrochloride;
3-(4-methoxyphenylamino)-*1H*-pyrazol-5-ol, or
4-hexylidene-3-phenyl-1-(pyridin-2-yl)-*1H*-pyrazol-5-ol; or a pharmaceutically acceptable salt thereof.

5. A compound selected from:
1-(pyridin-2-yl)-3-phenyl-4-propyl-*1H*-pyrazol-5-ol hydrochloride,
1-(pyridin-2-yl)-3-phenyl-4-propyl-*1H*-5-(isopropyloxycarbonyloxymethyloxy)pyrazole,
1-(pyridin-2-yl)-3-[(3-methoxy-4-hydroxyphenyl)-*E-*ethenyl]-4-benzyl-*1H*-pyrazol-5-ol hydrochloride,
1-(pyridin-2-yl)-3-[(3-methoxy-4-hydroxyphenyl)-E-ethenyl]-4-benzyl-*1H*-pyrazol-5-ol, or
1-(pyridin-2-yl)-3-phenyl-*1H*-pyrazol-5-ol hydrochloride; or a pharmaceutically acceptable salt thereof.

6. A method for preparing a compound represented by the following formula 1 or a pharmaceutically acceptable salt, the method comprising:
adding dropwise a compound represented by the following formula 2 and a compound represented by the following formula 3 to a polar organic solvent; and
heating the polar organic solvent comprising the compound represented by formula 2 and the compound represented by formula 3: 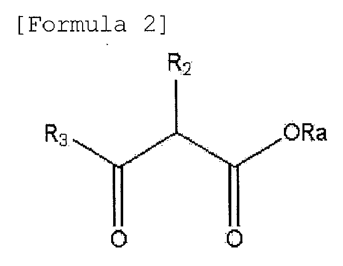 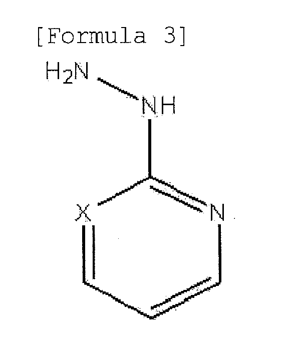 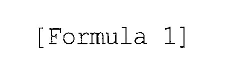 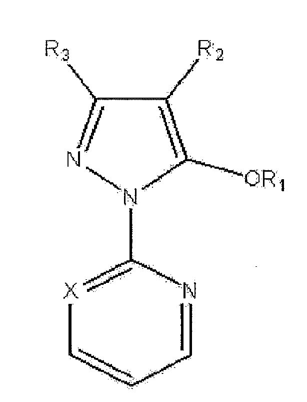
wherein X represents -CH- or nitrogen;
Ra represents a C1-C4 linear or branched alkyl;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₁ and R₂ each represents a hydrogen atom, R₃ represents a phenyl, a nitrophenyl, or a substituted or unsubstituted diphenylethenyl; and
wherein the substituent is a nitro, a hydroxyl, or a methoxyl.

7. The method of claim 6, wherein a compound represented by the following formula 4 is prepared by reaction of the compound represented by formula 2 and the compound represented by formula 3 in the presence of the organic solvent: 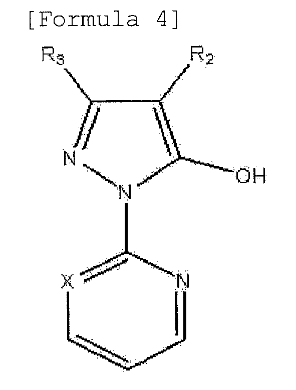 wherein X represents -CH- or nitrogen;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₂ represents a hydrogen atom, R₃ represents a phenyl, a nitrophenyl, or a substituted or unsubstituted diphenylethenyl; and
wherein the substituent is a nitro, a hydroxyl, or a methoxyl.

8. The method of claim 7, further comprising reacting the compound represented by formula 4 with at least one halide compound selected from the group consisting of isopropyloxycarbonyloxymethyliodide, isopropyloxycarbonyloxymethylchloride, and isopropyloxycarbonyloxymethylbromide in the presence of a base.

9. The method of claim 8, wherein the compound represented by formula 4 and the halide compound are reacted in the presence of a phase transfer catalyst.

10. The method of claim 6, wherein the polar organic solvent is selected from C1-C4 alcohol, acetic acid, and a mixture thereof.

11. The method of claim 6, wherein the adding is carried out at -4 °C to 10 °C.

12. The method of claim 6, wherein the heating is carried out at 90 °C to 130 °C.

13. The method of claim 8, wherein the base is at least one selected from the group consisting of 4-dimethylaminopyridine (DMAP), pyridine, triethylamine, imidazole, and carbonate.

14. The method of claim 6, wherein the compound represented by formula 1 or a pharmaceutically acceptable salt thereof is:
1-(pyridin-2-yl)-3-phenyl-4-propyl-*1H*-pyrazol-5-ol,
1-(pyridin-2-yl)-3-phenyl-4-benzyl-*1H*-pyrazol-5-ol,
1-(pyridin-2-yl)-3-phenyl-4-(4-nitrobenzyl)-*1H*-pyrazol-5-ol,
1-(pyridin-2-yl)-3-phenyl-4-propyl-*1H*-pyrazol-5-ol hydrochloride,
1-(pyridin-2-yl)-3-phenyl-4-propyl-*1H*-5-(isopropyloxycarbonyloxymethyloxy)pyrazole,
1-(pyridin-2-yl)-3-[(3-methoxy-4-hydroxyphenyl)-E-ethenyl]-4-benzyl-*1H*-pyrazol-5-ol hydrochloride,
3-(4-nitrophenyl)-3-[2-(pyrimidin-2-yl)hydrazono]-propionic acid ethyl ester,
1-(pyrimidin-2-yl)-3-(4-nitrophenyl)-5-*1H*-pyrazol-5-ol,
1-(pyridin-2-yl)-3-(1,2-diphenyl-E-ethenyl)-*1H*-pyrazol-5-ol,
1-(pyridin-2-yl)-3-phenyl-*1H*-pyrazol-5-ol hydrochloride,
1-(pyridin-2-yl)-3-[(3-methoxy-4-hydroxyphenyl)-E-ethenyl]-4-benzyl-*1H*-pyrazol-5-ol,
3-(4-methoxyphenylamino)-*1H*-pyrazol-5-ol, or
4-hexylidene-3-phenyl-1-(pyridin-2-yl)-*1H*-pyrazol-5-ol.

15. A composition for preventing or treating osteoporosis, comprising a compound represented by the following formula 1: 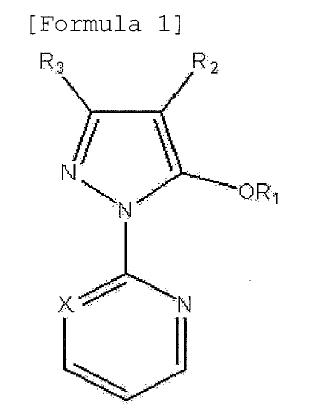 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when X represents -CH- and R₁ and R₂ each represents a hydrogen atom, R₃ represents a substituted or unsubstituted diphenylethenyl, or when X represents a nitrogen atom and R₁ and R₂ each represents a hydrogen atom, R₃ represents a nitrophenyl or a substituted or unsubstituted diphenylethenyl; and
wherein the substituent is a nitro, a hydroxyl, or a methoxyl.

16. A composition for preventing or treating osteoporosis, comprising a pharmaceutically acceptable salt of a compound represented by the following formula 1: 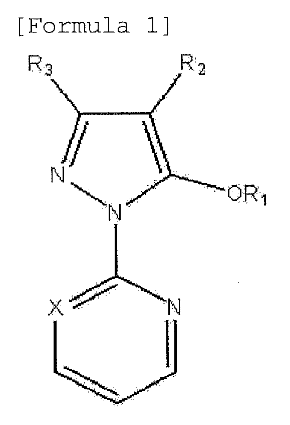 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₁ and R₂ each represents a hydrogen atom, R₃ represents a phenyl, a nitrophenyl, or a substituted or unsubstituted diphenylethenyl; and
wherein the substituent is a nitro, a hydroxyl, or a methoxyl.

17. A composition for preventing or treating osteoporosis, comprising at least one compound selected from:
1-(pyridin-2-yl)-3-phenyl-4-propyl-*1H*-pyrazol-5-ol,
1-(pyridin-2-yl)-3-phenyl-4-benzyl-*1H*-pyrazol-5-ol,
1-(pyridin-2-yl)-3-phenyl-4-(4-nitrobenzyl)-*1H*-pyrazol-5-ol,
1-(pyridin-2-yl)-3-phenyl-4-propyl-*1H*-pyrazol-5-ol hydrochloride,
1-(pyridin-2-yl)-3-phenyl-4-propyl-*1H*-5-(isopropyloxycarbonyloxymethyloxy)pyrazole,
1-(pyridin-2-yl)-3-[(3-methoxy-4-hydroxyphenyl)-E-ethenyl]-4-benzyl-*1H*-pyrazol-5-ol hydrochloride,
1-(pyridin-2-yl)-3-[(3-methoxy-4-hydroxyphenyl)-E-ethenyl]-4-benzyl-*1H*-pyrazol-5-ol,
3-(4-nitrophenyl)-3-[2-(pyrimidin-2-yl)hydrazono]-propionic acid ethyl ester,
1-(pyrimidin-2-yl)-3-(4-nitrophenyl)-5-*1H*-pyrazol-5-ol,
1-(pyridin-2-yl)-3-(1,2-diphenyl-E-ethenyl)-*1H*-pyrazol-5-ol,
1-(pyridin-2-yl)-3-phenyl-*1H*-pyrazol-5-ol hydrochloride,
3-(4-methoxyphenylamino)-*1H*-pyrazol-5-ol, and
4-hexylidene-3-phenyl-1-(pyridin-2-yl)-*1H*-pyrazol-5-ol;
and a pharmaceutically acceptable salt thereof.

18. A method for preventing or treating osteoporosis, the method comprising administering a compound represented by the following formula 1 to a mammal: 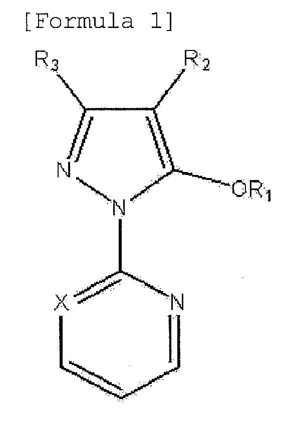 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when X represents -CH- and R₁ and R₂ each represents a hydrogen atom, R₃ represents a substituted or unsubstituted diphenylethenyl, or when X represents a nitrogen atom and R₁ and R₂ each represents a hydrogen atom, R₃ represents a nitrophenyl or a substituted or unsubstituted diphenylethenyl; and
wherein the substituent is a nitro, a hydroxyl, or a methoxyl.

19. A method for preventing or treating osteoporosis, the method comprising administering a pharmaceutically acceptable salt of a compound represented by the following formula 1 to a mammal: 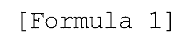 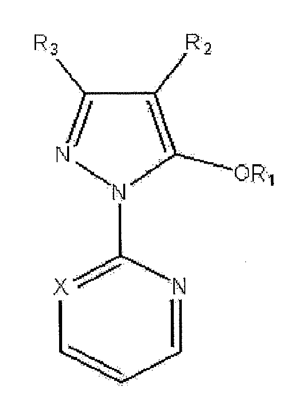 wherein X represents -CH- or nitrogen;
R₁ represents a hydrogen atom or an isopropyloxycarbonyloxymethyl;
R₂ represents a hydrogen atom, a C1-C4 linear or branched alkyl, or a substituted or unsubstituted benzyl; and
R₃ represents a phenyl, a nitrophenyl, a substituted or unsubstituted phenylethenyl, or a substituted or unsubstituted diphenylethenyl;
wherein when R₁ and R₂ each represents a hydrogen atom, R₃ represents a phenyl, a nitrophenyl, or a substituted or unsubstituted diphenylethenyl; and
wherein the substituent is a nitro, a hydroxyl, or a methoxyl.

20. A method for preventing or treating osteoporosis, the method comprising administering to a mammal at least one compound selected from:
1-(pyridin-2-yl)-3-phenyl-4-propyl-*1H*-pyrazol-5-ol,
1-(pyridin-2-yl)-3-phenyl-4-benzyl-*1H*-pyrazol-5-ol,
1-(pyridin-2-yl)-3-phenyl-4-(4-nitrobenzyl)-*1H*-pyrazol-5-ol,
1-(pyridin-2-yl)-3-phenyl-4-propyl-*1H*-pyrazol-5-ol hydrochloride,
1-(pyridin-2-yl)-3-phenyl-4-propyl-*1H*-5-(isopropyloxycarbonyloxymethyloxy)pyrazole,
1-(pyridin-2-yl)-3-[(3-methoxy-4-hydroxyphenyl)-E-ethenyl]-4-benzyl-*1H*-pyrazol-5-ol hydrochloride,
1-(pyridin-2-yl)-3-[(3-methoxy-4-hydroxyphenyl)-E-ethenyl]-4-benzyl-*1H*-pyrazol-5-ol,
3-(4-nitrophenyl)-3-[2-(pyrimidin-2-yl)hydrazono]-propionic acid ethyl ester,
1-(pyrimidin-2-yl)-3-(4-nitrophenyl)-5-*1H*-pyrazol-5-ol,
1-(pyridin-2-yl)-3-(1,2-diphenyl-E-ethenyl)-*1H*-pyrazol-5-ol,
1-(pyridin-2-yl)-3-phenyl-*1H*-pyrazol-5-ol hydrochloride,
3-(4-methoxyphenylamino)-*1H*-pyrazol-5-ol, and
4-hexylidene-3-phenyl-1-(pyridin-2-yl)-*1H*-pyrazol-5-ol; and a pharmaceutically acceptable salt thereof.
